# EUROPÄISCHE PATENTSCHRIFT

(11) **EP 1 004 430 B1**
(45) Veröffentlichungstag und Bekanntmachung des Hinweises auf die Patenterteilung: **18.02.2004**
(21) Anmeldenummer: 99123490.7
(22) Anmeldetag: 25.11.1999
(51) Int. Cl.: B30B 11/14, A61J 3/10, A61K 9/20

(54) **Vorrichtung und Verfahren zur Herstellung von Antierstickungspastillen**
Method and apparatus for manufacturing anti-choke tablets
Procédé et dispositif pour la fabrication de pastilles évitant la suffocation

(30) Priorität: 26.11.1998 IT MI982564
(43) Veröffentlichungstag der Anmeldung: 31.05.2000
(73) Patentinhaber: Zaini Luigi S.p.A., 20159 Milano (IT)
(72) Erfinder: Zaini, Luigi, 20122 Milano (IT)
(74) Vertreter: Radünz, Ingo, Dipl.-Ing.

(56) Entgegenhaltungen:
- WO-A-99/66905
- GB-A- 2 332 639
- US-A- 2 214 191
- US-A- 3 009 413
- US-A- 3 113 076
- US-A- 3 898 121
- US-A- 4 172 112
- US-A- 5 071 607
- US-A- 5 073 379
- PATENT ABSTRACTS OF JAPAN vol. 017, no. 164 (M-1390), 30. März 1993 (1993-03-30) & JP 04 327398 A (HITACHI POWDERED METALS CO LTD), 16. November 1992 (1992-11-16)

## Beschreibung

Pastillen mit jeweils einer zentralen Bohrung in der Mitte sind schon bekannt. Die bekannten Pastillen haben jedoch den Nachteil, daß sie keine ausreichende Belüftung der Lunge gewährleisten können, falls die Pastille zufällig in den Rachen-Luftröhren-Bereich gelangen sollte. In der nicht vorveröffentlichten WO 99/66905 ist eine zum Einnehmen vorgesehene Pastille beschrieben, die mit einem oder mehreren Kanälen versehen ist, um im Falle des Verschluckens eine Erstickung zu vermeiden.

Der Erfindung liegt die Aufgabe zugrunde, eine Vorrichtung zu entwickeln, die eine Herstellung von Antierstickungspastillen ermöglicht, die eine ausreichende Belüftung der Lunge auch dann sicherstellen, wenn die Pastille zufällig in den Rachen-Luftröhren-Bereich gelangen sollte, und ein Verfahren zu schaffen, das die spezielle Herstellung einer solchen Pastille gestattet.

Die Aufgabe wird durch eine Vorrichtung mit den Merkmalen des Anspruches 1 und ein Verfahren mit den Merkmalen des Anspruches 4 gelöst. Vorteilhafte Ausgestaltungen der Erfindung sind Gegenstand der Unteransprüche.

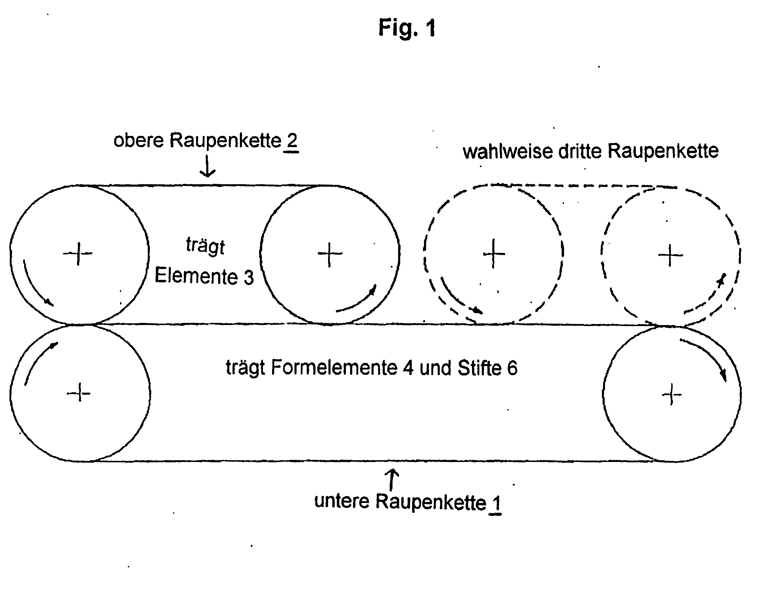

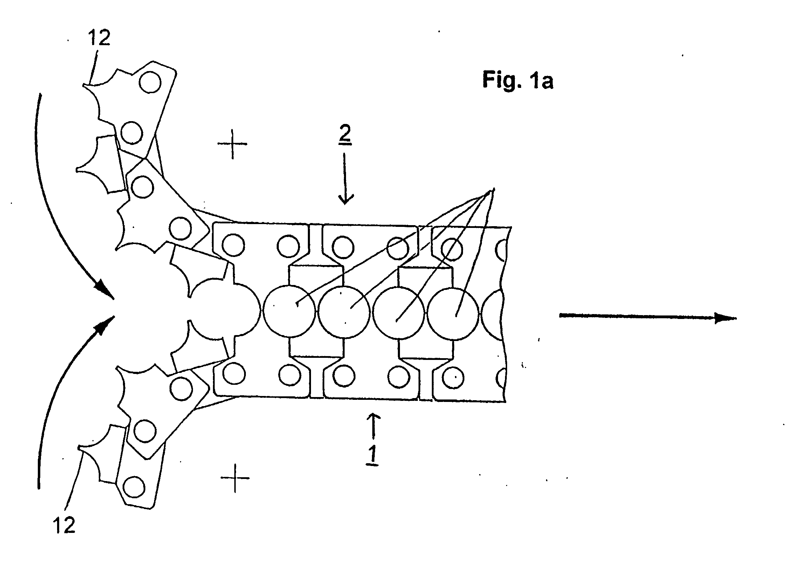

### Stand der Technik unter Bezugnahme auf Fig. 1

Der Produktionszyklus der Kristallpastillen oder harten Pastillen besteht im wesentlichen darin, daß in einem zerlegbaren Tunnel mit unterschiedlichem Querschnitt und unterschiedlicher Form wie in einem Rohr sich zwei Formstempel bewegen, die das innere Profil des Tunnels abbilden und in einer konvergierenden Bewegung das zuvor eingeführte Produktmaterial pressen und so die Pastille formen.

Danach zerlegt sich der Tunnel wieder und die Formstempel ziehen sich zurück, wodurch das geformte Produkt ausgestoßen wird.

Das System wird aus ineinandergreifenden und auf Antriebszahnrädern beweglichen Elementen gebildet - wie eine Raupenkette. Die Abschnitte der Raupenketten sind so übereinander angeordnet, daß der untere Teil des Abschnittes einer oberen Raupenkette 2 sich genau in das Oberteil des Abschnittes einer unteren Raupenkette 1 einpaßt.

Die miteinander in Kontakt stehenden Raupenkettenabschnitte bilden den Tunnel, in dem sich die Formstempel bewegen. Die Tunnel verlaufen längs des linearen Weges der Raupenkettenabschnitte und an ihrem Ende bewegen sich die Elemente, welche die Raupenketten bilden, längs der Antriebszahnräder nach oben (die des oberen Abschnitts) und nach unten (die des unteren Abschnitts); so wird der Kontakt zwischen den beiden Raupenkettenabschnitten unterbrochen und die Tunnel öffnen sich. Auf der entgegengesetzten Seite geschieht genau das Gegenteil, wobei die Ränder der die Tunnel bildenden Elemente in der Phase der Neubildung des Tunnels die Funktion von Messern haben, die das Produktmaterial auf das korrekte Maß zurechtschneiden.

Das Produktmaterial wird kontinuierlich durch ein übliches Speisesystem geliefert.

Die Fig. 1a zeigt einen Längsschnitt der unteren Raupenkette 1 und der oberen Raupenkette 2 gemäß dem Stand der Technik.

### Beschreibung der Erfindung

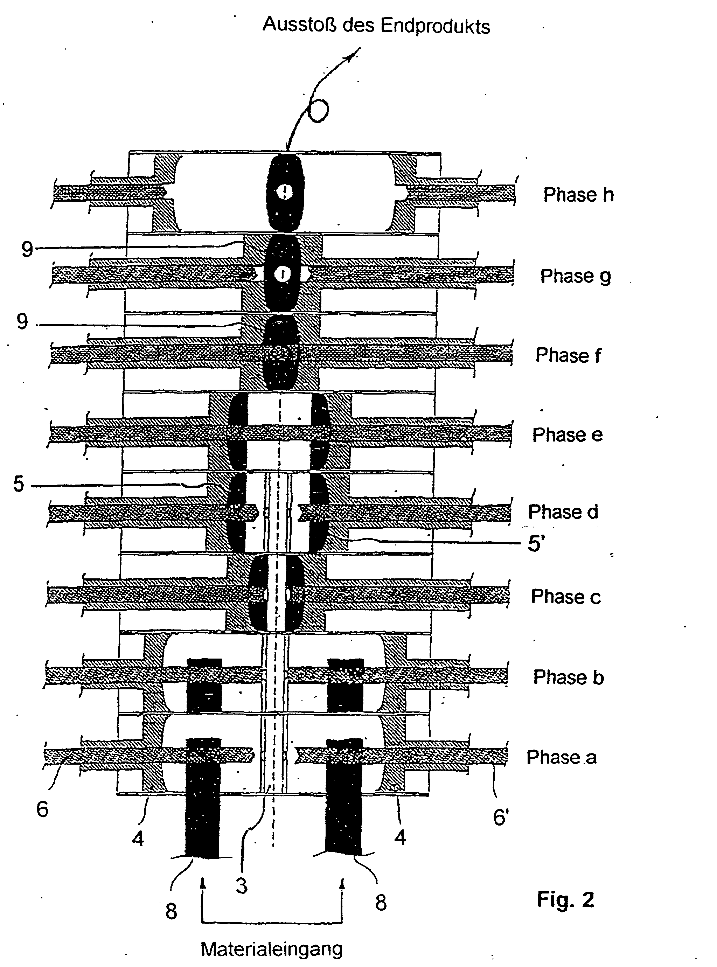

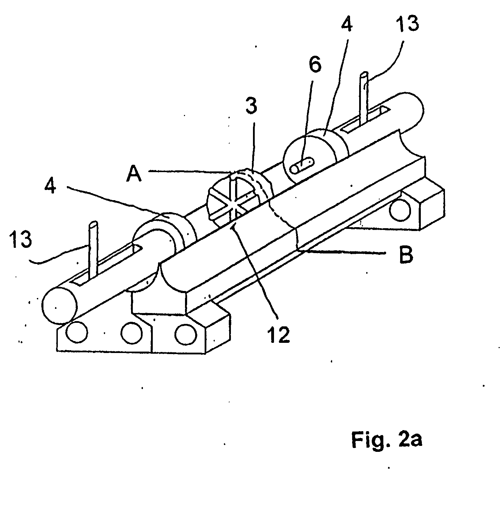

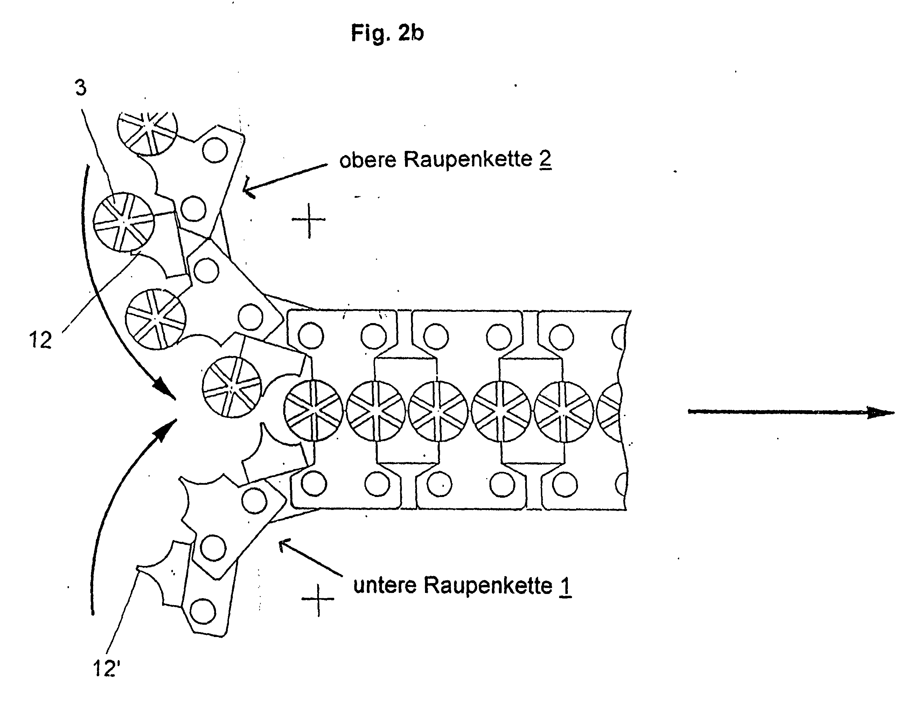

In Fig. 2 werden die Erfindung und die verschiedenen Phasen dargestellt, welche die Herstellung der mit einem Kanalsystem versehenen Pastillen ermöglichen.

Die Fig. 2a stellt das Element 3 dar, das am Messer 12 der oberen Raupenkette 2 befestigt ist.

Im Formstempel 4 befindet sich die Antriebsspindel 13 für die beweglichen Stifte 6, 6'.

Die Fig. 2b ist ein Längsschnitt durch die obere Raupenkette 2 und durch die untere Raupenkette 1 gemäß der Linie A - B der Fig. 2a.

Genauer gesagt, besteht die Erfindung darin, daß den Formstempeln 4 ein Element 3 zugeordnet ist, das als Gegenform zu den Formstempeln 4 dient. Dieses Gegenformelement 3 weist beidseitig eine geeignete Profilierung auf und ermöglicht es, die beiden Pastillenhälften 5, 5' mit den entsprechenden Rillen zu versehen. Sobald die beiden Pastillenhälften miteinander verbunden sind, weisen sie echte durchgehende Kanäle auf. Das Profil des Gegenformelements kann entsprechend verändert werden.

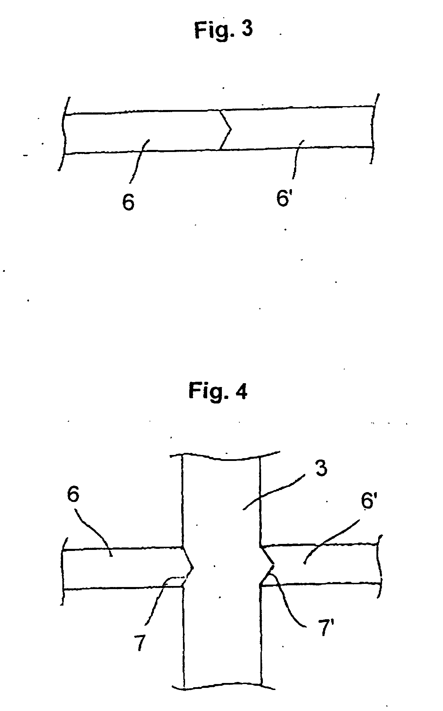

Die Formstempel 4 weisen ein oder zwei Längsbohrungen auf, in denen sich ein oder mehrere Stifte 6, 6' bewegen. Diese Stifte sind an einem Ende jeweils mit einem Profil versehen (Vorsprung und Ausnehmung), so daß die Profile in der Schließphase zusammenpassen und beim Miteinanderverbinden der beiden Pastillenhälften 5, 5' Stabilität gewährleisten (siehe Fig. 3).

Das Gegenformelement 3 weist keine Bohrung auf, sondern hat im Zentrum eine Ausnehmung 7 auf der einen Seite und einen Vorsprung 7' auf der anderen Seite, wobei die Ausnehmung 7 mit dem Vorsprung des Stiftes 6 und der Vorsprung 7' mit der Ausnehmung des Stiftes 6' zusammenpassen (siehe Fig. 4).

Wie das Gegenformelement 3 können auch die Formstempel 4 verschiedene Profile haben, je nach der Form, die man der Pastille verleihen will (z.B. rund, oval oder quadratisch). Auf jeden Fall ist immer eine zentrale Bohrung vorhanden, die unerläßlich ist.

Das Verfahren zur Herstellung von Pastillen mit einem Kanalsystem umfaßt folgende Phasen:

### Phase a

Die Tunnel werden teilweise geschlossen; das Gegenformelement 3 wird bei geöffneten Stiften 6, 6' in Stellung gebracht, und das Produktmaterial 8 wird eingeführt.

### Phase b

Die Stifte 6, 6' werden in eine geschlossene Stellung gebracht; und das Produktmaterial 8 wird auf das richtige Maß zugeschnitten.

### Phase c

Die Formstempel 4 werden in Richtung auf das Gegenformelement 3 zusammengefahren, und die beiden Pastillenhälften 5, 5' werden gepreßt.

### Phase d

Die Formstempel 4, die Stifte 6, 6' und die beiden aus dem Produktmaterial 8 gepreßten Pastillenhälften 5, 5' werden zurückgezogen und vom Gegenformelement 3 gelöst.

### Phase e

Die Stifte 6, 6' werden miteinander in Eingriff gebracht und das Gegenformelement 3 wird mit der oberen Raupenkette 2 gehoben.

### Phase f

Die Formstempel 4 werden so weit vorgeschoben, daß ein leichter Kontakt mit den beiden Pastillenhälften 5, 5' hervorgerufen wird, womit die Phase des Miteinanderverbindens der beiden Hälften bewerkstelligt wird.

### Phase g

Die Stifte 6, 6' werden zurückgezogen, so daß die zentrale Bohrung 11 der Pastille 9 hergestellt ist, und die verbundenen Pastillenhälften 5, 5' werden als Pastille 9 durch die Formstempel 4 festgehalten.

### Phase h

Die Formstempel 4 werden zurückgezogen und geben das fertige Produkt als Pastille 9 frei.

Durch Einführung des Elements 3 zusätzlich zu den Formstempeln 4 und der beweglichen Stifte 6, 6' im Inneren der Bohrung oder der Bohrungen der Formstempel 4 sowie der besonderen Verfahrensweise entsprechend den oben beschriebenen Phasen wird ermöglicht, Pastillen herzustellen, die mit einem System untereinander verbundener und nach außen führender Kanäle versehen sind.

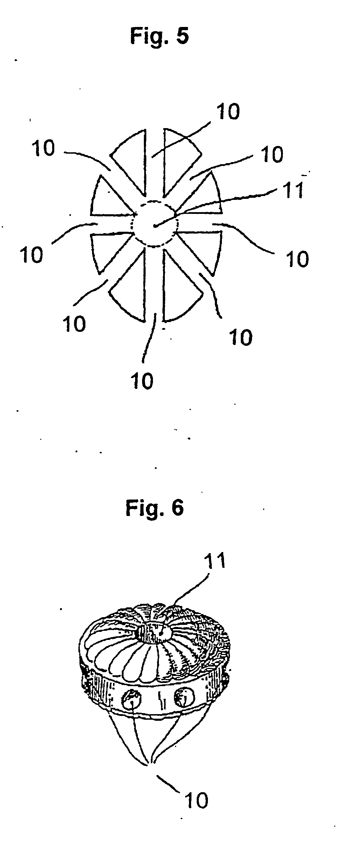

In Fig. 5 ist als Beispiel eine Pastille im Querschnitt mit dem Kanalsystem aus den Kanälen 10 und der zentralen Bohrung 11 dargestellt.

Fig. 6 zeigt eine perspektivisch dargestellte Antierstickungspastille, die mit der Vorrichtung und nach dem Verfahren wie oben beschrieben hergestellt wurde, mit der zentralen Bohrung 11 und den Mündungen der Kanäle 10.

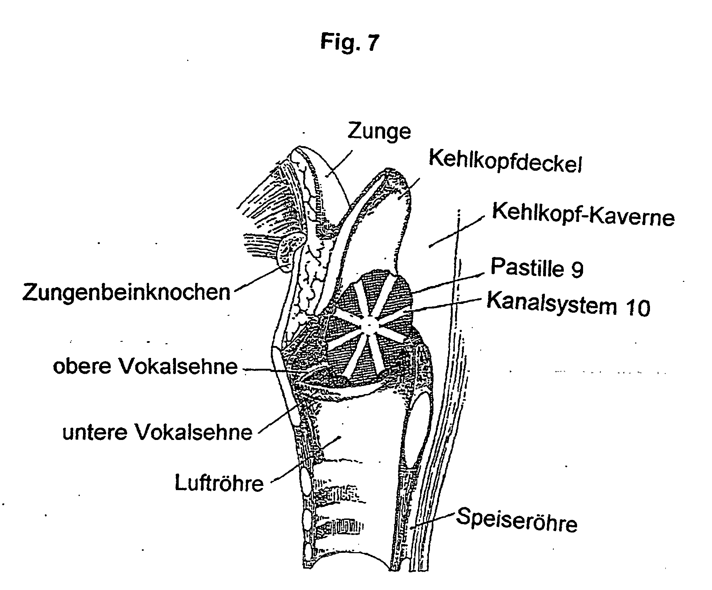

Fig. 7 ist ein Schnitt durch den Rachen-Luftröhren-Raum, in der eine mit den Kanälen 10 gemäß der Erfindung eingeklemmte Pastille 9 gezeigt ist, die eine ausreichende Belüftung der Lunge gewährleistet, wenn die Pastille zufällig in den Rachen-Luftröhren-Raum gelangen sollte.

Alle mit Hilfe der Vorrichtung und des Verfahrens der Erfindung hergestellten Pastillen 9, gleichgültig ob sie rund, oval oder viereckig sind, haben miteinander gemeinsam, daß sie mit einem System von untereinander verbundenen und nach außen führenden Kanälen 10 sowie einer durchgehenden zentralen Bohrung 11 versehen sind.

## Patentansprüche

1. Vorrichtung für die Herstellung von Antierstickungspastillen, **dadurch gekennzeichnet, daß** zwei Formstempel (4) eine oder mehrere Längsbohrungen aufweisen, in denen bewegliche Stifte (6, 6') angeordnet sind, und daß ein Element (3) zentral zwischen den beiden Formstempeln (4) zugeordnet ist, das als Gegenform dient und beidseitig mit einer Profilierung versehen ist, die es ermöglicht, die beiden Pastillenhälften (5, 5') mit Rillen zu versehen, die nach der Verbindung der Pastillenhälften (5, 5') durchgehende Kanäle (10) bilden.

2. Vorrichtung nach Anspruch 1, **dadurch gekennzeichnet, daß** das Element (3) als Gegenformelement ausgebildet ist und in der Mitte auf der einen Seite einen Vorsprung (7) und auf der anderen Seite eine Ausnehmung (7') aufweist.

3. Vorrichtung nach Anspruch 2, **dadurch gekennzeichnet, daß** die Stifte (6, 6') an einem Ende mit einem Profil versehen sind, das als Vorsprung oder als Ausnehmung ausgebildet ist.

4. Verfahren zur Herstellung von Antierstickungspastillen mit einer Vorrichtung nach einem der Ansprüche 1 bis 3, bei dem eine untere und eine obere Raupenkette (1, 2) einen Tunnel bilden, der entlang eines linearen Weges sich schließt und wieder öffnet, **gekennzeichnet durch** folgende Phasen:
- Phase a: in den sich schließenden Tunnel werden das Gegenformelement (3) und das Produktmaterial (8) eingeführt;
- Phase b: die Stifte (6, 6') in den Formstempeln (4) werden gegen das Gegenformelement (3) bewegt, und das Produktmaterial (8) wird auf das richtige Maß zugeschnitten;
- Phase c: die Formstempel (4) werden in Richtung auf das Gegenformelement (3) zusammengefahren, und aus dem Produktmaterial (8) werden die beiden Pastillenhälften (5, 5') gepresst;
- Phase d: die Formstempel (4), die Stifte (6, 6') und die beiden gepreßten Pastillenhälften (5, 5') werden zurückgezogen und vom Gegenformelement (3) gelöst;
- Phase e: die Stifte (6, 6') werden miteinander in Eingriff gebracht, und das Gegenformelement (3) wird mit der oberen Raupenkette (2) gehoben und damit vom Raum zwischen den beiden Formstempeln (4) entfernt;
- Phase f: die Formstempel (4) werden so weit vorgeschoben, daß ein leichter Kontakt mit den beiden Pastillenhälften (5, 5') hervorgerufen wird, womit die Phase des Miteinanderverbindens der beiden Pastillenhälften (5, 5')bewerkstelligt wird;
- Phase g: die Stifte (6, 6') werden zurückgezogen und die zentrale Bohrung (11) der Pastille (9) ist hergestellt, und das verbundene Produkt als Pastille (9) wird **durch** die Formstempel (4) festgehalten;
- Phase h: die Formstempel (4) werden zurückgezogen, und das fertige Produkt als Pastille (9) wird freigegeben.

## Claims

1. Device for the production of anti-asphyxiation pastilles, **characterised in that** two moulding dies (4) have one or more longitudinal bores, in which movable pins (6, 6') are arranged, and that an element (3) is associated centrally between the two moulding dies (4) and serves as countermould, the element being provided at both sides with a profiling which makes it possible to provide the two pastille halves (5, 5') with grooves which after connection of the pastille halves (5, 5') form continuous channels (10).

2. Device according to claim 1, **characterised in that** the element (3) is formed as a countermoulding element and has in the centre a projection (7) on one side and a recess (7') on the other side.

3. Device according to claim 2, **characterised in that** the pins (6, 6') are provided at one end with a profile which is formed as a projection or as a recess.

4. Method of producing anti-asphyxiation pastilles by a device according to one of claims 1 to 3, in which a lower and an upper track chain (1, 2) form a tunnel, which close and open again along a linear path, **characterised by** the following phases:
- phase a: the countermoulding element (3) and the product material (8) are introduced into the closing tunnel;
- phase b: . the pins (6, 6') are moved into the moulding dies (4) against the countermoulding element (3) and the product material (8) is cut to the correct dimension;
- phase c: the moulding dies (4) are moved together in direction towards the countermoulding element (3) and the two pastille halves (5, 5') are press-moulded from the product material (8);
- phase d: the moulding dies (4), the pins (6, 6') and the two press-moulded pastille halves (5, 5') are drawn back and released from the countermoulding element (3);
- phase e: the pins (6, 6') are brought into engagement with one another and the countermoulding element (3) is raised together with the upper track chain (2) and thus removed from the space between the two moulding dies (4);
- phase f: the moulding dies (4) are advanced to such an extent that a light contact with the two pastilles halves (5, 5') is produced, whereby the phase of interconnection of the two pastille halves (5, 5') is brought about;
- phase g: the pins (6, 6') are drawn back and the central bore (11) of the pastille (9) is produced and the connected product as a pastille (9) is retained by the moulding dies (4); and
- phase h: the moulding dies (4) are drawn back and the finished product, as a pastille (9), is released.

## Revendications

1. Dispositif pour la fabrication de pastilles anti-étouffantes, **caractérisé en ce que** deux poinçons de forme (4) comportent un ou plusieurs trous longitudinaux, dans lesquels sont disposées des broches mobiles (6, 6'), et qu'un élément (3) est adjoint centralement entre les deux poinçons de forme (4), qui sert de forme contraire et qui est doté des deux côtés d'un profilage qui permet de doter les deux moitiés de pastilles (5, 5') de rainures, lesquelles forment des canaux traversants (10) après l'assemblage des moitiés de pastilles (5, 5').

2. Dispositif selon la revendication 1, **caractérisé en ce que** l'élément (3) est configuré comme élément de forme contraire et comporte au milieu d'un côté une saillie (7) et de l'autre côté un évidement (7').

3. Dispositif selon la revendication 2, **caractérisé en ce que** les broches (6, 6') sont dotées à une extrémité d'un profil qui est configuré comme saillie ou comme évidement.

4. Procédé pour la fabrication de pastilles anti-étouffantes avec un dispositif selon l'une des revendications 1 à 3, dans lequel une chenille inférieure et une chenille supérieure (1, 2) forment un tunnel, qui se ferme et s'ouvre de nouveau le long d'un trajet linéaire, **caractérisé par** les phases suivantes :
- Phase a : l'élément de forme contraire (3) et la matière du produit (8) sont introduits dans le tunnel qui se ferme ;
- Phase b : les broches (6, 6') dans les poinçons de forme (4) sont déplacées contre l'élément de forme contraire (3), et la matière du produit (8) est coupée à la bonne mesure ;
- Phase c : les poinçons de forme (4) sont rapprochés en direction de l'élément de forme contraire (3), et les deux moitiés de pastilles (5, 5') sont pressées dans la matière du produit (8) ;
- Phase d : les poinçons de forme (4), les broches (6, 6') et les deux moitiés de pastilles (5, 5') pressées sont retirés et dégagés de l'élément de forme contraire (3) ;
- Phase e : les broches (6, 6') sont amenées en prise les unes avec les autres et l'élément de forme contraire (3) est soulevé avec la chenille supérieure (2) et est ainsi éloigné de l'espace entre les deux poinçons de forme (4) ;
- Phase f : les poinçons de forme (4) sont avancés suffisamment pour qu'un léger contact avec les deux moitiés de pastilles (5, 5') soit provoqué, par.lequel la phase de l'assemblage des deux moitiés de pastilles (5, 5') est réalisée ;
- Phase g : les broches (6, 6') sont retirées et le trou central (11) de la pastille (9) est réalisé, et le produit assemblé en tant que pastille (9) est maintenu par les poinçons de forme (4) ;
- Phase h : les poinçons de forme (4) sont retirés et le produit fini est libéré en tant que pastille (9).
